# EUROPEAN PATENT APPLICATION

(11) **EP 1 582 164 A1**
(43) Date of publication of application: **05.10.2005**
(21) Application number: 04007790.1
(22) Date of filing: 31.03.2004
(51) Int. Cl.: A61B 17/72

(54) **Intramedullary nail comprising a stem whereon longitudinal portions are provided driving elements of shape-memory material**

(71) Applicant: ORTHOFIX INTERNATIONAL B.V., 1071 Amsterdam (NL)
(72) Inventor: Coati, Michele, 37029 San Pietro in Cariano (Verona) (IT); Marazzi, Giancarlo, 21047 Saronno (Varese) (IT); Marini, Graziano, 37060 Castel D'Azzano (Verona) (IT); Rossi, Graziano, 37139 Verona (IT); Rossi, Luigi, 37019 Peschiera de Garda (Verona) (IT); Venturini, Daniele, 37064 Povegliano Veronese (Verona) (IT)
(74) Representative: Ferreccio, Rinaldo

(57) **Abstract**

An intramedullary nail (10, 110, 210, 310) suitable for insertion in a fractured elongate bone (12) and for an unusually simple fixation therein comprises a substantially straight stem (14), having a predetermined axis (X-X), extending between a proximal end (16) and a distal end (18) and expansion means (20) for the nail fixation to the bone, said expansion means (20) being operable by at least one driving element (22) realised with a shape-memory material.

## Description

### Field of application

The present invention relates in its more general aspect to an intramedullary nail suitable for insertion in a fractured elongate bone and an application method of said nail in said bone.

In particular, the invention relates to an intramedullary nail suitable for insertion in a fractured elongate bone, such as a femur or a tibia, comprising a substantially straight stem, having a predetermined axis, extending between a proximal end and a distal end.

### Prior art

Intramedullary nails are known, which, during a surgical operation, are inserted in a fractured elongate bone and fixed therein, in order to reconstruct the original bone configuration and meanwhile recover the bone solidity, so that callus regeneration mechanisms can correctly occur.

The stems of these intramedullary nails are generally cylinder-shaped and they can be both solid and hollow.

In order to fix the intramedullary nail to the bone portions to be reconstructed, two offset holes are usually provided on the nail, having axes lying on parallel planes and extending diametrically across the stem, in correspondence with the nail distal end, and two offset holes having the same size, having axes not necessarily lying on parallel planes, in correspondence with the nail proximal end. Said holes are suitable for housing bone screws, which are inserted, after a convenient bone drilling, in the bone, with the subsequent fixation of the intramedullary nail to the bone portions.

Although advantageous under different points of view, intramedullary nails being structured as above schematically described have known drawbacks mainly underlined when bone drillings are to be performed for bone screw insertion. This step is particularly critical since it is known that a good nail fastening essentially depends on the correct realisation of these bone drillings, obviously made in correspondence with the holes of the inserted intramedullary nail.

However the precise location of the intramedullary nail holes is made difficult by the fact that the holes are no more visible, being the nail inserted in the bone. It is then worth underlining a further location problem, i.e. the fact that the intramedullary nail can be, when being inserted, slightly bent, so that the holes at the nail distal end are no more, with respect to the proximal end, in the same position as before installing the nail.

The traditional technique for locating the holes of an inserted intramedullary nail provides the use of X rays, involving however the well known risks of cumulative exposure of the operating staff, besides being quite awkward during the surgical operation.

Specific mechanical devices have thus been studied and realised, such as for example the one described in the European patent no. EP 772 420 in the name of the same Applicant.

These devices do not require the use of X rays, but they have the drawback of requiring several operational steps, all to be performed quite carefully and precisely.

### Summary of the invention

The problem underlying the present invention is to provide an intramedullary nail suitable for insertion in a fractured elongate bone, capable to meet the above-mentioned requirement, meanwhile overcoming, in a simple and effective way, all the drawbacks mentioned with reference to the prior art.

This problem is solved, according to the present invention, by an intramedullary nail suitable for insertion in a fractured elongate bone, as above described and characterised in that it comprises expansion means for the nail fixation to the bone, said expansion means being operable by at least one driving element realised with a shape-memory material.

Further features and the advantages of the intramedullary nail suitable for insertion in a fractured elongate bone, as well as of the application method of said nail in said fractured bone, according to the present invention, will be apparent from the following description of an embodiment thereof made with reference to the attached drawings, given by way of non-limiting example.

### Brief description of the drawings

Figure 1 is a schematic perspective exploded view of an portion of an intramedullary nail, suitable for insertion in a fractured elongate bone, according to the present invention.
Figure 2 is a schematic side-elevation view of the nail of figure 1, in a different configuration.
Figure 3 is a schematic plan view from above of the nail of figure 2.
Figure 4 schematically shows a detail of the nail of figure 1.
Figure 5 schematically shows a detail of the nail of figure 2.
Figure 6 is a schematic plan view from above of a first alternative embodiment of a nail according to the present invention.
Figure 7 schematically shows a cross-section of the nail of figure 6, taken according to the traced VII-VII plane of figure 6.
Figure 8 is a schematic plan view from above of the nail of figure 6, in a different configuration.
Figure 9 schematically shows a cross-section of the nail of figure 8, taken according to the traced IX-IX plane of figure 8.
Figure 10 is a schematic perspective view of a portion of a second alternative embodiment of the intramedullary nail according to the present invention.
Figure 11 is a schematic perspective view of a third alternative embodiment of an intramedullary nail according to the present invention.
Figure 12 is a schematic perspective view of the intramedullary nail of figure 12, in a different configuration.

### Detailed description

With reference to the drawings, an intramedullary nail according to the present invention is shown in a first embodiment and in three further alternative embodiments.

The nail, in the embodiment described at first, is globally indicated with 10, while in the alternative embodiments the nail of the invention is indicated with 110, 210 and 310 respectively. It is specified that, in these alternative embodiments, the elements being structurally or functionally similar to the elements of the nail 10 are indicated with the same reference number and the detailed description thereof is not repeated for short.

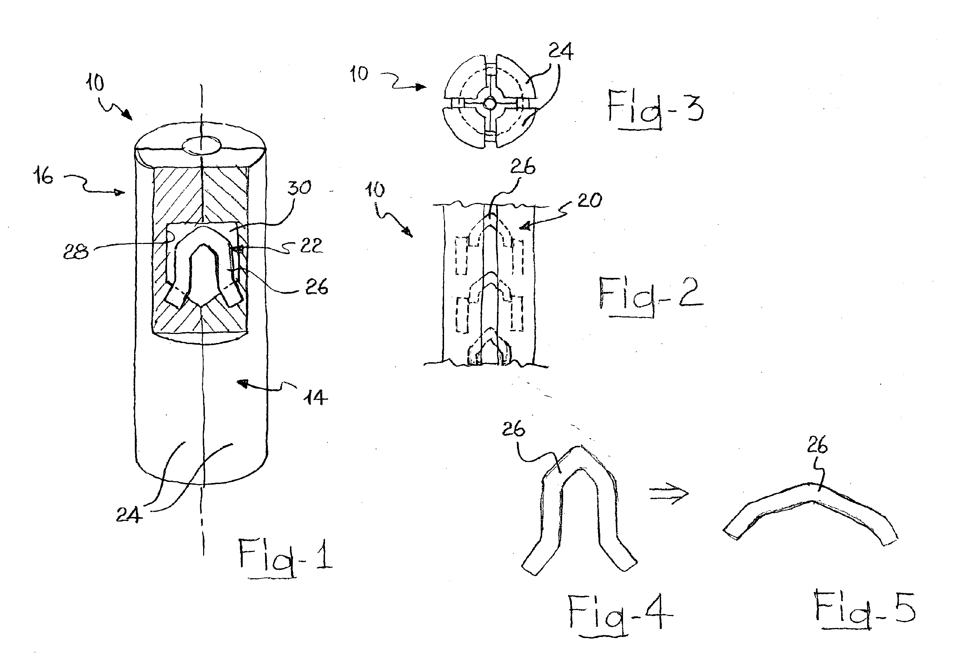

The nail 10 shown in figures 1, 2 and 3, which is suitable for insertion in a fractured elongate bone 12, such as for example a femur or a tibia, comprises a substantially straight stem 14, having a predetermined axis X-X, extending between a proximal end 16 and a distal end 18.

According to an aspect of the present invention, the nail 10 comprises expansion means 20 for the nail fixation to the bone, said expansion means 20 being operable by at least one driving element 22 realised with a shape-memory material.

Shape memory material means a material having a given initial starting shape and taking, under predetermined external conditions (for example a temperature rise and/or drop) or undergoing a predetermined activation condition, i.e. after a so-called "material instruction" step, a given new shape.

Known shape-memory materials suitable for use in the present invention are, for instance, certain nickel-titanium alloys.

The invention is based on the following principle: subjecting an element realised in a shape memory material, having a predetermined initial shape at rest, to said so-called "material instruction" step (for example to a predetermined temperature variation), said element takes a shape being maintained as long as the "instruction" step effect persists; said shape, being different from the initial shape, can be called transient shape and it is temporary or unstable. When the "instruction" step effect stops, the element leaves said transient shape and, coming back towards the initial shape, takes a working shape. Depending on the type of material and on the working temperature, in the working shape, said material can arrive at the initial shape or it can go further on the initial shape, arriving at a final shape. It is worth pointing out that the more this final shape is far from the initial shape, the more the available shape memory energy of the material is high.

The invention has been reached as result of the intuition that an element realised in a shape memory material, in the passage from the transient shape towards the working shape, can create the fixing of the intramedullary nail in the bone.

In the example of figures 1, 2 and 3, the stem 14 comprises a plurality of longitudinal portions 24 forming, close to each other, a cylindrical tubular body.

In other words, the longitudinal portions 24 have a circular-crown-sector-shaped section. In the case being shown, the longitudinal portions 24 are four and the angle at the circular crown sector centre is a right angle.

Expansion means 20 shape in said longitudinal portions 24, which can be radially spaced apart from the axis X-X of the stem 14.

Said at least one driving element 22, for example in the shape of a V-or-U-bent cylindrical body 26, is provided between the longitudinal portions 24. The cross section of the cylindrical body 26 can be, for example, circular or square.

The cylindrical body 26 is fixed, at the two opposite ends, to two longitudinal portions 24, generally in correspondence with surfaces being turned towards the axis X-X.

Preferably, recesses 28, being accessible from the axis X-X, are provided in said longitudinal portions 24. Said recesses 28 of the longitudinal portions 24 are conjugate to each other: in other words, when the longitudinal portions 24 are close to each other, said recesses 28 define an area 30 comprising said bent cylindrical body 26 which is connected, at two opposite ends, to the two longitudinal portions 24 being concerned.

In the case of the four longitudinal portions 24 being shown, four cylindrical bodies 26 are provided in correspondence with each end 16 and 18 of the stem 14. Preferably, the four cylindrical bodies 26 of each end 16 and 18 are symmetrically positioned with respect to the axis X-X, generally at a same height with respect to the axis X-X.

In the initial shape, shown in figure 1, the longitudinal portions 24 are close to each other and the cylindrical bodies 26 are bent.

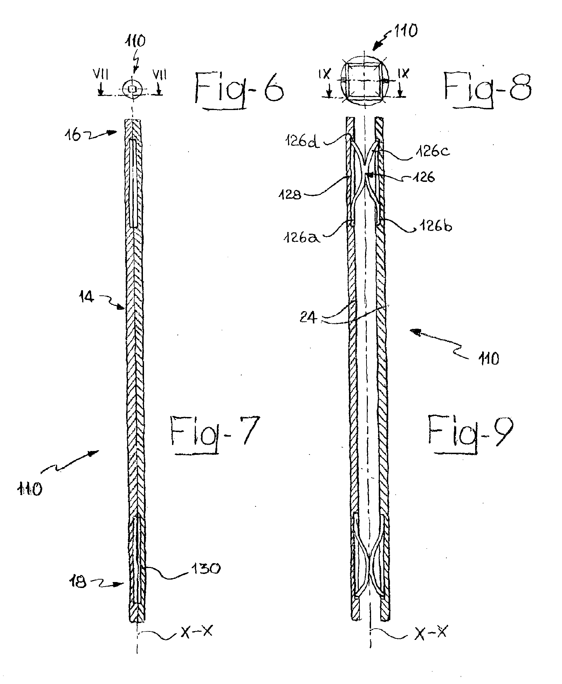

In the final shape, shown in figures 2 and 3, i.e. when the shape-memory material of the cylindrical bodies 26 is activated, the cylindrical bodies 26 tend to straighten, i.e. the angle between the directions of two opposite ends tends to increase, as it can be seen by comparing figures 4 and 5 showing a cylindrical body 26 in the initial shape and in the final shape. As a consequence of this straightening, the longitudinal portions 24, connected by means of the cylindrical bodies 26, are spaced apart, having moved in a substantially radial direction with respect to the axis X-X.

In the alternative embodiment of figures 6, 7, 8 and 9, the nail 110 according to the invention is shown, wherein at least one driving element 22, shaping in a X-shaped structure 126, is provided between the longitudinal portions 24.

The structure 126 has thus four ends 126a, 126b, 126c and 126d: in correspondence with two first adjacent ends 126a and 126b, the structure 126 is fixed to two longitudinal portions 24, generally in correspondence with surfaces being turned towards the axis X-X. The fixation is performed for example through buckling with plastic deformation.

Preferably, grooves 128, being accessible from the axis X-X, are provided in said longitudinal portions 24. Said grooves 128 of the longitudinal portions 24 are conjugate to each other: in other words, when the longitudinal portions 24 are close to each other, said grooves 128 define an area 130 comprising said structure 126 when being bent, i.e. when the two first adjacent ends 126a and 126b are close to each other and when also the two remaining adjacent ends 126c and 126d are close to each other.

The two remaining ends 126c and 126d are slideble in said grooves 128.

In the case being shown, the longitudinal portions 24 are four and four structures 126 are provided in correspondence with each end 16 and 18 of the stem 14. Preferably, the four structures 126 of each end 16 and 18 are symmetrically positioned with respect to the axis X-X, generally at a same height with respect to the axis X-X.

In the initial shape, shown in figures 6 and 7, the longitudinal portions 24 are close to each other and the structures 126 are bent as above described.

In the final shape, shown in figures 8 and 9, i.e. when the shape-memory material of the structures 126 is activated, the first two adjacent ends 126a and 126b tend to space apart, as the remaining adjacent ends 126c and 126d tend to space apart by sliding in the grooves 128, i.e. the structures 126 become X-shaped. As a consequence of this straightening, the longitudinal portions 24, connected by means of the structures 126, are spaced apart, having moved in a substantially radial direction with respect to the axis X-X.

It must be noticed that the X-shaped structures 126 can be alternatively obtained by joining pairs of V-or-U-bent cylindrical bodies 26, in correspondence with the bent area. It is evident that in this case, in order to achieve the same effect as structures 126, the cylindrical bodies 26 will be so instructed as to be bent in the final shape and to be substantially straight in the initial shape.

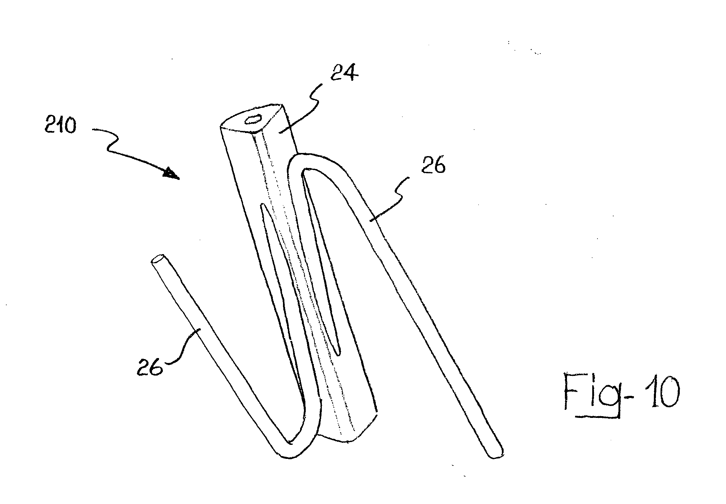

In the alternative embodiment of figure 10, the nail 210 according to the invention is shown, which is similar to the nail 10 and it has thus V-or-U-bent cylindrical bodies 26. However, in this alternative embodiment the adjacent cylindrical bodies 26 are positioned with the bend arranged on opposite sides: in other words, in the case of four longitudinal portions 24 with four cylindrical bodies 26, the cylindrical bodies 26, which face to each other, are specular.

It must be noticed that the radial expansion is linked to the length of the cylindrical bodies 26.

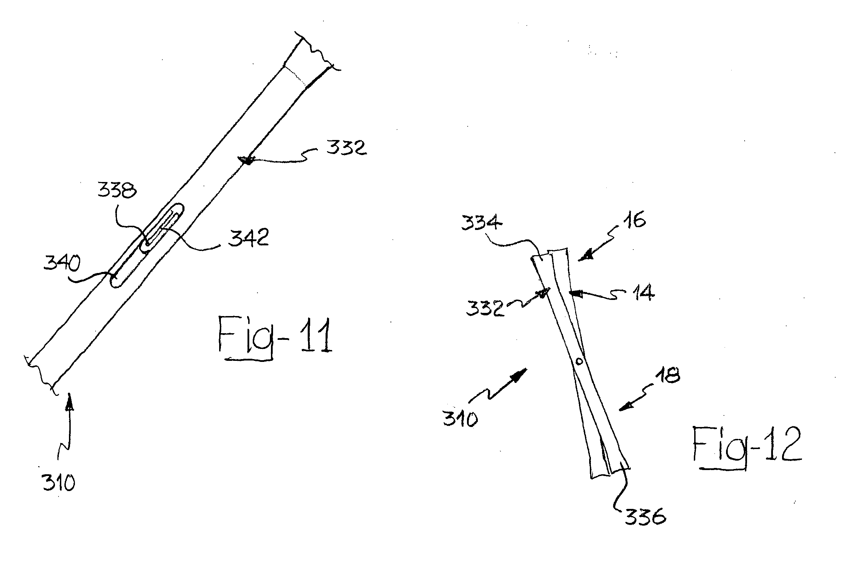

In the alternative embodiment of figures 11 and 12, the nail 310 according to the invention is shown, wherein expansion means 20 shape in a rod 332, two opposite ends 334 and 336 of said rod 332 being spaceable in a substantially radial way from the axis X-X of the stem 14.

More precisely, the rod 332 is hinged, in an intermediate position thereof, on the stem 14 by means of a pin 338. In particular, the rod 332 is hinged in an intermediate position of the stem 14.

The rod 332 is rectangle-shaped and substantially as long as the stem 14. The rod 332 is also equipped with a groove 340, in correspondence with the area wherein the pin 338 is positioned. A torsion spring 342 realised with a shape-memory material is positioned around the pin 338: this is the driving element 22 of the nail 310.

An end of the spring 342 is constrained to the stem 14, while the opposite end is positioned in front of a wall of the groove 340, or preferably it is connected thereto.

In the final shape, the spring 342 is expanded, with an increase in the angle between the two ends of the spring 342 causing, through the thrust of the opposite end of the spring 342 on the wall of the groove 340, a rotation of the rod 332.

The rod 332 is preferably equipped, at the two ends 334 and 336, with sharp portions for an improved fixation of the nail 310.

An application method of an intramedullary nail according to the present invention in said fractured elongate bone 12 comprises:
a positioning step of said nail in said elongate bone 12, to mend the fracture;
an operation step of said expansion means 20 for the nail fixation to the bone, said expansion means 20 being operated by at least one driving element 22 realised with a shape-memory material.

The operation of the intramedullary nail 10, suitable for insertion in a fractured elongate bone 12, according to the present invention, is already partially evident from the description of the above application method and it is specified hereafter.

The nail according to the invention is positioned in said elongate bone 12, in the initial shape thereof.

Afterwards, in order to fix the nail to the bone 12, said driving element 22 realised with a shape-memory material is operated.

In the first three embodiments, the longitudinal portions 24 radially space apart up to realise an interference with the medullary canal surfaces surrounding the longitudinal portions 24.

This interference practically causes a grip, solidly fixing the nail in the bone 12.

In the forth embodiment, considering that the stem 14 can be very long, the ends 334 and 336 of the rod 332 space apart in a substantially radial way from the axis X-X of the stem 14.

These ends 334 and 336 realise an interference with the bone portion surrounding them. This interference practically causes a grip, solidly fixing the nail in the bone 12.

The main advantage achieved by the intramedullary nail suitable for insertion in a fractured elongate bone, as well as by the application method of said nail in said fractured bone, according to the present invention, is the fact of unusually simplifying the fixation step of the intramedullary nail inserted in the fractured bone. In practise, the nail fixation is obtained simultaneously with the insertion thereof in the bone, since the shape-memory elements, cooled at first to take the initial shape, take in the bone their final shape, due to the heat released by the patient's body.

Another advantage of the intramedullary nail according to the present invention, is to prevent undesired nail torsions or rotations in the bone during the application step.

For the first three embodiments, it must be noticed how advantageously the network formed by the cylindrical bodies 26 or by the X-shaped structures 126 and by the longitudinal portions 24 gives an excellent structural resistance to the nail of the invention.

In the case of the forth embodiment, it must be noticed how advantageously the torsion spring 342 ensures a considerable rotation of the rod 332.

Obviously, in order to meet specific and contingent requirements, a skilled in the art could bring several changes to the above-described intramedullary nail suitable for insertion in a fractured elongate bone and application method of said nail in said bone, all however comprised in the scope of protection of the present invention, as defined in the following claims.

## Claims

1. Intramedullary nail (10, 110, 210, 310) suitable for insertion in a fractured elongate bone (12), comprising a substantially straight stem (14), having a predetermined axis (X-X), extending between a proximal end (16) and a distal end (18), **characterised in that** it comprises expansion means (20) for the nail fixation to the bone, said expansion means (20) being operable by at least one driving element (22) realised with a shape-memory material.

2. Intramedullary nail (10, 110, 210) according to claim 1, **characterised in that** the stem (14) comprises a plurality of longitudinal portions (24) forming, close to each other, a cylindrical tubular body.

3. Intramedullary nail (10, 110, 210) according to claim 2, **characterised in that** the longitudinal portions (24) are four.

4. Intramedullary nail (10, 110, 210) according to claim 2, **characterised in that** expansion means (20) shape in said longitudinal portions (24), which can be radially spaced apart from the axis (X-X) of the stem (14).

5. Intramedullary nail (10, 110, 210) according to claim 2, **characterised in that** said at least one driving element (22) is provided between the longitudinal portions (24).

6. Intramedullary nail (10, 210) according to claim 5, **characterised in that** said at least one driving element (22) is in the shape of a V-or-U-bent cylindrical body (26).

7. Intramedullary nail (10, 110, 210) according to claim 6, **characterised in that** the cylindrical body (26) is fixed, at the two opposite ends, to two longitudinal portions (24).

8. Intramedullary nail (10, 110, 210) according to claim 7, **characterised in that** the cylindrical body (26) is fixed in correspondence with surfaces being turned towards the axis (X-X) of the stem (14).

9. Intramedullary nail (10, 110) according to claim 8, **characterised in that** recesses (28), being accessible from the axis (X-X) of the stem (14), are provided in said longitudinal portions (24), said recesses (28) of the longitudinal portions (24) being conjugate to each other, so as to define, when the longitudinal portions (24) are close to each other, an area (30) comprising said bent cylindrical body (26) which is connected, at two opposite ends, to said two longitudinal portions (24).

10. Intramedullary nail (10, 210) according to claims 3 and 6, **characterised in that** four cylindrical bodies (26) are provided in correspondence with each end (16, 18) of the stem (14).

11. Intramedullary nail (10, 210) according to claim 10, **characterised in that** the four cylindrical bodies (26) of each end (16, 18) are symmetrically positioned with respect to the axis (X-X) of the stem (14).

12. Intramedullary nail (10, 210) according to claim 11, **characterised in that** the four cylindrical bodies (26) of each end (16, 18) are positioned at a same height with respect to the axis (X-X) of the stem (14).

13. Intramedullary nail (10, 210) according to claim 6, **characterised in that**, in the shape preceding the nail fixation, the longitudinal portions (24) are close to each other and the cylindrical bodies (26) are bent.

14. Intramedullary nail (10, 210) according to claim 6, **characterised in that**, when the shape-memory material of the cylindrical bodies (26) is activated, the cylindrical bodies (26) tend to straighten.

15. Intramedullary nail (110) according to claim 2, **characterised in that** at least one driving element (22), shaping in a X-shaped structure (126) is provided between the longitudinal portions (24).

16. Intramedullary nail (110) according to claim 15, **characterised in that**, in correspondence with two first adjacent ends (126a, 126b) of the structure (126), the structure (126) is fixed to two longitudinal portions (24).

17. Intramedullary nail (110) according to claim 16, **characterised in that** the structure (126) is fixed in correspondence with surfaces being turned towards the axis (X-X) of the stem (14).

18. Intramedullary nail (110) according to claim 17, **characterised in that** the fixation of the structure (126) is performed through buckling with plastic deformation.

19. Intramedullary nail (110) according to claim 16, **characterised in that** grooves (128), being accessible from the axis (X-X) of the stem (14), are provided in said longitudinal portions (24), said grooves (128) being conjugate to each other so as to define, when the longitudinal portions (24) are close to each other, an area (130) comprising said structure (126) when being bent, i.e. when said two first adjacent ends (126a, 126b) are close to each other and when also the two remaining adjacent ends (126c, 126d) are close to each other.

20. Intramedullary nail (110) according to claim 19, **characterised in that** said two remaining ends (126c, 126d) are slideble in said grooves (128).

21. Intramedullary nail (110) according to claim 15, **characterised in that** the longitudinal portions (24) are four and four structures (126) are provided in correspondence of each end (16, 18) of the stem (14).

22. Intramedullary nail (110) according to claim 21, **characterised in that** the four structure (126) of each end (16, 18) are symmetrically positioned with respect to the axis (X-X) of the stem (14).

23. Intramedullary nail (110) according to claim 22, **characterised in that** the four structures (126) of each end (16, 18) are positioned at a same height with respect to the axis (X-X) of the stem (14).

24. Intramedullary nail (110) according to claim 15, **characterised in that**, in the shape preceding the nail fixation, the longitudinal portions (24) are close to each other and the structures (126) are bent.

25. Intramedullary nail (110) according to claim 20, **characterised in that**, when the shape-memory material of the structures (126) is activated, the first two adjacent ends (126a, 126b) tend to space apart, as the remaining adjacent ends (126c, 126d) tend to space apart by sliding in the grooves (128).

26. Intramedullary nail (110) according to claim 15, **characterised in that** the X-shaped structures (126) are obtained by joining pairs of V-or-U-bent cylindrical bodies (26), in correspondence with the bent area.

27. Intramedullary nail (210) according to claim 6, **characterised in that** the adjacent cylindrical bodies (26) are positioned with the bend arranged on opposite sides.

28. Intramedullary nail (310) according to claim 1, **characterised in that** expansion means (20) shape in a rod (332), two opposite ends (334, 336) of said rod (332) being spaceable in a substantially radial way from the axis (X-X) of the stem (14).

29. Intramedullary nail (310) according to claim 28, **characterised in that** the rod (332) is hinged, in an intermediate position thereof, on the stem (14) by means of a pin (338).

30. Intramedullary nail (310) according to claim 29, **characterised in that** the rod (332) is hinged in an intermediate position of the stem (14).

31. Intramedullary nail (310) according to claim 30, **characterised in that** the rod (332) is rectangle-shaped and substantially as long as the stem (14).

32. Intramedullary nail (310) according to claim 29, **characterised in that** the rod (332) is equipped with a groove (340) in correspondence with the area wherein the pin (338) is positioned (340).

33. Intramedullary nail (310) according to claim 32, **characterised in that** a torsion spring (342), realised with a shape-memory material and being the driving element (22) of the nail (310), is positioned around the pin (338).

34. Intramedullary nail (310) according to claim 33, **characterised in that** an end of the spring (342) is constrained to the stem (14), while the opposite end is positioned in front of a wall of the groove (340).

35. Intramedullary nail (310) according to claim 33, **characterised in that** an end of the spring (342) is constrained to the stem (14), while the opposite end is connected to a wall of the groove (340).

36. Intramedullary nail (310) according to claims 34 or 35, **characterised in that**, when the shape-memory material of the spring (342) is activated, the spring (342) is expanded, with an increase in the angle between the two ends of the spring (342).

37. Intramedullary nail (310) according to claim 28, **characterised in that** the rod (332) is equipped at the two ends (334, 336) with sharp portions.

38. Intramedullary nail (10, 110, 210, 310) according to claim 1, **characterised in that** the fractured elongate bone (12) is a femur or a tibia.

39. Application method, in a fractured elongate bone (12), of an intramedullary nail (10, 110, 210, 310) according to claim 1, **characterised in that** it comprises:
a positioning step of said nail (10, 110, 210, 310) in said elongate bone (12), to mend the fracture;
an operation step of said expansion means (20) for the nail fixation to the bone, said expansion means (20) being operated by at least one driving element (22) realised with a shape-memory material.
